# EUROPEAN PATENT SPECIFICATION

(11) **EP 3 194 981 B1**
(45) Date of publication and mention of the grant of the patent: **22.08.2018**
(21) Application number: 16729171.5
(22) Date of filing: 03.06.2016
(51) Int. Cl.: G01N 33/68

(54) **OBSTRUCTIVE SLEEP APNEA (OSA) BIOMARKER PANEL**
OBSTRUKTIVER SCHLAFAPNOE (OSA) BIOMARKER-PANEL
GROUPE DE BIOMARQUEURS D'APNÉE OBSTRUCTIVE DU SOMMEIL (OSA)

(30) Priority: 05.06.2015 US 201562171754 P
(43) Date of publication of application: 26.07.2017
(73) Proprietor: Beckman Coulter, Inc., Brea, CA 92821 (US)
(72) Inventor: SOUTHWICK, Paula C., San Diego, CA 92126 (US); LU, Jiuliu, Palmetto Bay, FL 33157 (US); RILEY, John S, Miami, FL 33196 (US); SAMOSZUK, Michael K., Rancho Santa Margarita, CA 92688 (US); CRUZ, Amabelle B., San Diego, CA 92127 (US)
(74) Representative: Boult Wade Tennant LLP
(86) International application number: PCT/US2016/035900
(87) International publication number: WO 2016/197053

(56) References cited:
- WO-A1-2007/051258
- WO-A2-2008/057966
- US-A1- 2014 072 959
- RASHID NADEEM ET AL: "Serum Inflammatory Markers in Obstructive Sleep Apnea: A Meta-Analysis", JOURNAL OF CLINICAL SLEEP MEDICINE, 15 October 2013 (2013-10-15), XP055300711, US ISSN: 1550-9389, DOI: 10.5664/jcsm.3070
- CAMILA HIROTSU ET AL: "Association Between Uric Acid Levels and Obstructive Sleep Apnea Syndrome in a Large Epidemiological Sample", PLOS ONE, vol. 8, no. 6, 24 June 2013 (2013-06-24), page e66891, XP055301012, DOI: 10.1371/journal.pone.0066891
- S Imagawa ET AL: "Levels of vascular endothelial growth factor are elevated in patients with obstructive sleep apnea--hypopnea syndrome", Blood, 15 August 2001 (2001-08-15), pages 1255-1257, XP055301042, United States DOI: 10.1182/blood.V98.4.1255 Retrieved from the Internet: URL:http://www.bloodjournal.org/content/bl oodjournal/98/4/1255.full.pdf [retrieved on 2016-09-08]
- Nikolaos Papanas ET AL: "Vascular Health and Risk Management", Vascular Health and Risk Management, vol. 5 1 January 2009 (2009-01-01), pages 751-756, XP055301033, Retrieved from the Internet: URL:https://www.dovepress.com/hba1c-is-ass ociated-with-severity-of-obstructive-sleep -apnea-hypopnea--peer-reviewed-article-VHR M [retrieved on 2016-09-08]
- FLEMING WESLEY ELON ET AL: "Blood biomarkers of endocrine, immune, inflammatory, and metabolic systems in obstructive sleep apnea", CLINICAL BIOCHEMISTRY, ELSEVIER INC, US, CA, vol. 49, no. 12, 13 May 2016 (2016-05-13), pages 854-861, XP029668252, ISSN: 0009-9120, DOI: 10.1016/J.CLINBIOCHEM.2016.05.005

## Description

### FIELD OF THE INVENTION

This invention relates to methods to aid the diagnosis and management of obstructive sleep apnea.

### BACKGROUND OF THE INVENTION

Obstructive sleep apnea (OSA) is a common disorder, characterized by repetitive episodes of complete (apnea) or partial (hypopnea) obstructions of the upper airway during sleep, with decreasing oxygen saturation and sleep fragmentation. More than 22 million American adults have OSA. In the Wisconsin Sleep Cohort Study, representing a large, random sample of 30 to 60 year old individuals reporting habitual snoring, 9% of women and 24% of men had OSA.

The World Health Organization estimates 100 million worldwide have OSA, and up to 90% of individuals with OSA remain undiagnosed. OSA prevalence is increasing and may soon become the most common chronic disease in industrialized countries.

Untreated OSA can lead to serious health consequences, including increased mortality. Recurrent respiratory events and hypoxemia cause sympathetic activation, hypertension, oxidative stress, and metabolic dysregulation. Patients with OSA have an elevated risk of developing coronary artery disease, cardiac arrhythmia, myocardial infarction, heart failure, stroke, diabetes, obesity, metabolic syndrome, and memory decline. OSA increases cardiovascular risks independent of factors such as age, sex, race, smoking, diabetes, obesity, dyslipidemia, and hypertension. In addition, individuals with untreated OSA are more likely to be involved in work-related or driving accidents.

Given the significant health issues associated with untreated OSA and the substantial healthcare costs in treating these OSA - associated morbidities that encompasses the central nervous systems and many other organ systems, early diagnosis of this treatable disorder is critical. Continuous positive airway pressure (CPAP) treatment reduces the risk of adverse outcomes.

Current diagnostic techniques such as questionnaires perform poorly. Definitive diagnostic sleep study testing (overnight polysomnography) is expensive, time-consuming, and uncomfortable. Consequently, patients are often not referred for this definitive testing. WO2007/051258 describes that the combination of IL-6 and VEGF may be useful in the diagnosis of OSA.

### BRIEF SUMMARY OF THE INVENTION

The invention is defined in the claims. This invention provides algorithms of combinations of biomarkers that can aid the diagnosis and treatment of patients having OSA with high degree of accuracy. In some embodiments, the algorithms are used in conjunction with polysomnography (sleep study) findings and clinical signs and symptoms, such as BMI, Age, Diastolic BP Systolic BP, and questionnaires such as the Epworth Sleepiness Scale, to determine the presence of and the severity of OSA in patients. In some embodiments, the algorithms of the combinations of biomarkers are used to monitor the effectiveness of a form of treatment for OSA.

In one aspect, the invention provides a method of diagnosing obstructive sleep apnea (OSA) in a patient. The method comprises: measuring the levels of HbAlc and at least one biomarker selected from CRP, IL-6, uric acid and EPO in a sample from a patient, the; and diagnosing the patient as having OSA if the measured level of the biomarkers are elevated. In one embodiment, the method is used to diagnose moderate/severe OSA.

The methods may employ a predetermined algorithm which is a combination of biomarkers, wherein the combination is Linear Model - Linear Value, Linear Model - Log Value, Non-linear Model - Linear Value, or Non-linear Model - Log Value combination.

The biomarkers may be selected such that the AUC of the method using the combined biomarkers in diagnosing OSA is at least 0.8.

The biomarkers may be selected such that the sensitivity of the method of using the combined biomarkers in diagnosing OSA is at least 80% and the specificity of the method is at least 60%. The biomarkers may be selected such that the sensitivity of the method of using the combined biomarkers in diagnosing OSA is at least 85% and the specificity of the method is at least 50%.

In one embodiment, the combination of biomarkers comprise HbAlc and CRP. In one embodiment, the combination of biomarkers further comprise EPO, IL-6, or uric acid.

In one embodiment, the biomarkers are a combination of two or three biomarkers selected from the combinations listed in Table 5.

The predetermined algorithm may be a Linear Model - Log Value combination of HbA1c, CRP, and EPO, represented by the mathematical formula: A*log(HbA1c) + B*log(CRP) + C*log(EPO).

The predetermined algorithm may be a Non-Linear Model-Linear Value combination of HbAlc, IL-6, and EPO.

In one aspect, the invention provides a method of determining whether a therapy is effective for treating OSA. The method comprises: measuring the levels of HbAlc and at least one or two biomarkers in the sample from the patient before therapy, and the at least one or two biomarkers are selected from the group of, CRP, IL-6, uric acid, and EPO; measuring the levels of the at least two or three biomarkers in a sample taken from a patient after the therapy. The therapy is effective if the post-treatment levels of the at least two or three biomarkers are decreased relative to the pre-treatment levels. This may be where a multimarker index is lower than the pre-treatment multimarker index and the predetermined algorithm is positive logic. The therapy is also effective if the multimarker index is higher than the predetermined reference value and the predetermined algorithm is negative logic.

In one aspect, the invention provides a method of determining whether a therapy is effective for treating OSA. The method comprises the steps of: measuring the levels of HbAlc and at least one or two biomarkers that are selected from the group consisting of CRP, IL-6, uric acid, and EPO in a sample from a patient at a time point after the therapy; comparing the measured levels of the at least two or three biomarkers against a predetermined reference value. The therapy is effective if the levels of the at least two or three biomarkers are lower than the predetermined reference value. This may be where a post-treatment multimarker index is lower than a predetermined reference value for the multimarker index for the two or more biomarkers and the predetermined algorithm is positive logic. The therapy is effective if the multimarker index is higher than the predetermined reference value and the predetermined algorithm is negative logic.

The predetermined algorithm used to determine whether the therapy is effective may be a combination of the biomarkers and the combination is Linear Model - Linear Value, Linear Model - Log Value, Non-linear Model - Linear Value, or Non-linear Model - Log Value combination.

In one aspect, the invention provides a non-transitory computer readable medium that has computer-executable instructions, which, when executed, causes a processor to perform a method of the invention. A diagnosis of OSA can be made if the multimarker index is higher than a predetermined reference value for that multimarker index and the predetermined algorithm is positive logic. A diagnosis of OSA can also be made if the multimarker index is lower than the predetermined reference value and the predetermined algorithm is negative logic.

The predetermined algorithm may be a combination of biomarkers, and the combination is Linear Model - Linear Value, Linear Model - Log Value, Non-linear Model - Linear Value, or Non-linear Model - Log Value combination. The biomarkers may be selected such that the AUC of the method of using the combination of the two or more biomarkers in diagnosing OSA is at least 0.8.
the methods of the invention may be computer implemented methods.

The biomarkers may be selected such that the sensitivity of the method of using the combined biomarkers in diagnosing OSA is at least 80% and the specificity of the method is at least 60%. The biomarkers may be selected such that the sensitivity of the method of using the combined biomarkers in diagnosing OSA is at least 85% and the specificity of the method is at least 50%.

In one embodiment, the two or more biomarkers comprise HbAlc and CRP. In one embodiment, the combination of biomarkers further comprise EPO, IL-6, or uric acid.

In one embodiment, the biomarkers are a combination of two or three biomarkers selected from the combinations listed in Table 5.

The predetermined algorithm may be a Linear Model - Log Value combination of HbAlc, CRP, and EPO, represented by the mathematical formula: A*log(HbA1c) + B*log(CRP) + C*log(EPO).

The predetermined algorithm may be a Non-Linear Model-Linear Value combination of HbAlc, CRP, and EPO.

In one aspect, the invention provides a computer system for diagnosing OSA comprising: a) a detection device configured to detect HbAlc and one or more biomarkers that are selected from the group consisting of CRP IL-6, uric acid, and EPO ; and b) an analyzing device comprising one or more processors and a non-transitory computer readable medium of the invention.

The system may further comprise a display device for the diagnosis. The display device indicates the patient has OSA if one or more multimarker indices produced by the one or more processors are higher than their respective predetermined reference values and the predetermined algorithm is positive logic. The display device also indicates the patient as having OSA if the multimarker index is lower than the predetermined reference value and the predetermined algorithm is negative logic.

Measuring the levels of CRP, IL-6 or EPO can be performed using an immunological assay. Measuring the level of HbAlc can be performed using a method involving both an immunological assay and a non-immunological assay; and measuring the level of uric acid can be performed using a non-immunological assay.

### DEFINITIONS

As used herein, the term "OSA" or obstructive sleep apnea refers to sleep disordered breathing (SDB), sleep-related breathing disorder (SRBD) and obstructive sleep apnea syndrome (OSAS).

As used herein, the term "subject" or "patient" generally refers to one who is to be tested, or has been tested for prediction, assessment, monitoring or diagnosis of OSA. The subject may have been previously assessed or diagnosed using other methods, such as those described herein or those in current clinical practice, or maybe selected as part of a general population (a control subject).

As used herein, the term "biomarker" refers to a biological molecule found in blood, other body fluids, or tissues that is a sign of a normal or abnormal processes, or of a condition relating to OSA. Biomarkers can be hormones, cytokines, polypeptides, peptides, proteins, protein isoforms, metabolites, and also mutated proteins, which play roles in at least one biological process, for example, endocrine or metabolic pathways. For purpose of this disclosure, biomarkers are molecules whose expression levels are changed in subjects who have OSA, including one or more molecules selected from the group consisting of HbAlc, CRP, IL-6, uric acid, and EPO.

As used herein, the term "analyze" includes determining a value or set of values associated with a sample by measurement of analyte levels in the sample. "Analyze" may further comprise and compare the levels against constituent levels in a sample or set of samples from the same subject or other subject(s). The biomarkers of the present teachings can be analyzed by any of various conventional methods known in the art. Some such methods include but are not limited to: measuring serum protein or sugar or metabolite or other analyte level, measuring enzymatic activity, and measuring gene expression.

As used herein, "clinical parameters" refer to all non-sample or non-analyte biomarkers of subject health status or other characteristics, such as, without limitation, blood pressure, body weight, height, and calculation of body mass index (BMI), Epworth Sleepiness Scale (ESS), used to assess Daytime sleepiness, and apnea-hypopnea index (AHI), used to diagnose and assess the severity of sleep disordered breathing. Apnea-hypopnea index measures the average number of apneas and hypopneas per hour of sleep. Apnea is defined as absence of airflow for 10 seconds or more; and hypopnea defined as a reduction in airflow associated with at least a 4% decrease in oxygen saturation persisting for at least 10 seconds.

As used herein, the term "statistically different" refers to that an observed alteration is greater than what would be expected to occur by chance alone (e.g., a "false positive"). Statistical significance can be determined by any of various methods well-known in the art. An example of a commonly used measure of statistical significance is the p-value. The p-value represents the probability of obtaining a given result equivalent to a particular datapoint, where the datapoint is the result of random chance alone. A result is often considered significant (not random chance) at a p-value less than or equal to 0.05.

As used herein, the term "accuracy" refers to the degree that a measured or calculated value conforms to its actual value. "Accuracy" in the clinical diagnosis of OSA relates to the proportion of actual outcomes (true positives or true negatives, wherein a subject is correctly classified as having OSA or as not having OSA, respectively. True positive (TP), means positive test result that accurately reflects the tested-for activity. For example in the context of the present invention a TP, is for example but not limited to, truly classifying a person having OSA as such. True negative (TN), means negative test result that accurately reflects the tested-for activity. For example in the context of the present invention a TN, is for example but not limited to, truly classifying a subject not having OSA as such. False negative (FN), means a result that appears negative but fails to reveal a situation. For example in the context of the present invention a FN, is for example but not limited to, falsely classifying a subject having OSA as not having OSA. "FP" is false positive, means test result that is erroneously classified in a positive category. For example in the context of the present invention, a FP, is for example but not limited to, falsely classifying a healthy subject as having OSA.

As used herein, the term "performance" relates to the overall usefulness and quality of a diagnostic or prognostic test using the biomarkers disclosed herein for OSA. The performance of a test is reflected by a number of parameters, such as, clinical and analytical accuracy, other analytical and process characteristics, such as use characteristics (e.g., stability, ease of use), health economic value, and relative costs of components of the test. Any of these factors may be the source of superior performance and thus usefulness of the test. One of the most important consideration for performance is accuracy of the test, which can be measured by appropriate "performance metrics," such as AUC.

As used herein, the term "sensitivity" refers to the true positive fraction of disease subjects. Sensitivity can be defined as the number of true positive samples divided by the sum of true positive and false negative samples, i.e., TP/(TP+FN). A sensitivity of 1 means that the test recognizes all diseased subjects, but does not connote how reliably the test recognizes healthy subjects.

As used herein, the term "specificity" refers to the true negative fraction of non-diseased or normal subjects. Specificity can be defined by number of true negative samples divided by the sum of true negative and false positive samples, i.e., TN/(TN+FP). A specificity of 1 means that a test recognizes all healthy subjects as being healthy, i.e., no healthy subject is identified as having the disease in question, but does not connote how reliably the test recognizes diseased subjects.

As used herein, the term "AUC" refers to "area under the curve" or C-statistic, which is examined within the scope of ROC (receiver-operating characteristic) curve analysis. AUC is an indicator that allows representation of the sensitivity and specificity of a test, assay, or method over the entire range of test (or assay) cut points with just a single value. Thus, AUC is an effective measurement of the quality of a biomarker or a combination of biomarkers for the purpose of the diagnosis. An AUC of an assay is determined from a diagram in which the sensitivity of the assay on the ordinate is plotted against 1-specificity on the abscissa. A higher AUC indicates a higher accuracy of the test; an AUC value of 1 means that all samples have been assigned correctly (specificity and sensitivity of 1), an AUC value of 0.5 means that the samples have been assigned with guesswork probability and the parameter thus has no significance.

Using AUCs through the ROC curve analysis to evaluate the accuracy of a diagnostic test are well known in the art, for example, as described in, Pepe et al., "Limitations of the Odds Ratio in Gauging the Performance of a Diagnostic, Prognostic, or Screening Marker," Am. J. Epidemiol 2004, 159 (9): 882-890, and "ROC Curve Analysis: An Example Showing The Relationships Among Serum Lipid And Apolipoprotein levels In Identifying Subjects With Coronary Artery Disease," Clin. Chem., 1992, 38(8): 1425-1428. See also, CLSI Document EP24-A2: Assessment of the Diagnostic Accuracy of Laboratory Tests Using Receiver Operating Characteristic Curves; Approved Guideline - Second Edition. Clinical and Laboratory Standards Institute; 2011; CLSI Document I/LA21-A2: Clinical Evaluation of Immunoassays; Approved Guideline - Second Edition. Clinical and Laboratory Standards Institute; 2008.

As used herein, the term "multimarker index": a multimarker index used in the invention is generated by assigning an algorithm to a combination of two or more biomarkers to provideboth qualitative diagnosis of OSA and quantitative measure of the severity of OSA in a subject. The algorithm is developed by applying various classification models based on a dataset of levels of multiple markers that are individually correlated to OSA. Classification models that can be used for this purpose are known in the art, including but are not limited to, Linear Model, Non-linear Model, Linear DA, Quadratic DA, Naive Bayes, linear regression, Quadratic Regression, KNN, Linear SVM, SVM with 2^{nd}-order polynomial Kernel, SVM with 3^{rd}-order Polynomial Kernel, Neural Networks, Parzen Windows, Fuzzy Logic, Decision Trees.

As used herein, the term "positive logic" means that a higher value of the multimarker index produced by a particular algorithm indicates a higher possibility of OSA. The term "negative logic" means that a lower value of the multimarker index produced by a particular algorithm indicates a higher possibility of OSA.

As used herein, the term "diagnosis" means the process of knowledge gaining by assigning symptoms or phenomena to a disease or injury. For the purpose of this invention, diagnosis means determining the presence of, and optionally, the severity of, OSA in a subject. The term "diagnosis" as used herein also refers to "screening".

As used herein, the term "prognosis" refers to is a prediction as to whether OSA is likely to develop in a subject. Prognostic estimates are useful in, e.g., determining an appropriate therapeutic regimen for a subject.

As used herein, the term "algorithm" encompasses any formula, model, mathematical equation, algorithmic, analytical or programmed process, or statistical technique or classification analysis that takes one or more inputs or parameters, whether continuous or categorical, and calculates an output value, index, index value or score. Examples of algorithms include but are not limited to ratios, sums, regression operators such as exponents or coefficients, biomarker value transformations and normalizations (including, without limitation, normalization schemes that are based on clinical parameters such as age, gender, ethnicity, etc.), rules and guidelines, statistical classification models, and neural networks trained on populations. Also of use in the context of biomarkers are linear and non-linear equations and statistical classification analyses to determine the relationship between (a) levels of biomarkers detected in a subject sample and (b) the presence (diagnosis of OSA) or severity of the respective subject's OSA.

As used herein, the term "predetermined reference value" or "reference value" refers to a threshold level of a biomarker or a threshold value of a multimarker index - generated by combining more than one biomarkers in a predetermined algorithm,- by comparing with which, a diagnosis of OSA can be made. The reference value can be a threshold value or a reference range. In one embodiment, a reference value can be derived from ROC curve analysis, selecting the reference value as that which maximizes sensitivity while keeping the specificity above a user-defined threshold. The reference value can also be selected as that which maximizes specificity while keeping the sensitivity above a user-defined threshold, for example, 80% sensitivity. In another embodiment, a reference value can be the upper limit of the range of a biomarker levels or of a multimarker indices produced from a population of healthy subjects, if the biomarker or multimarker index is increased in subjects having OSA, i.e., the predetermined algorithm is positive logic,. Conversely, a reference value can be the lower limit of the range of a biomarker levels or of a multimarker indices produced from a population of healthy subjects, if the biomarker or multimarker index is decreased in subjects having OSA, i.e., the algorithm is negative logic,.

A "therapeutic regimen," "therapy" or "treatment(s)," as described herein, includes all clinical management of a subject and interventions, whether biological, chemical, physical, or a combination thereof, intended to sustain, ameliorate, improve, or otherwise alter the condition of OSA in a subject. These terms may be used synonymously herein. Treatments include but are not limited to lifestyle changes such as losing weight or quitting smoking, continuous positive airway pressure (CPAP) therapy, oral appliances designed to keep the throat open, surgery, administration of prophylactics or therapeutic compounds, exercise regimens, physical therapy, dietary modification and/or supplementation, bariatric surgical intervention, administration of pharmaceuticals and/or anti-inflammatories (prescription or over-the-counter), and any other treatments known in the art as efficacious in preventing, delaying the onset of, ameliorating or curing OSA. A "response to treatment" includes a subject's response to any of the above-described treatments, whether biological, chemical, physical, or a combination of the foregoing. A "course of treatment" relates to the dosage, duration, extent, etc. of a particular treatment or therapeutic regimen.

### BRIEF DESCRIPTION OF THE DRAWINGS

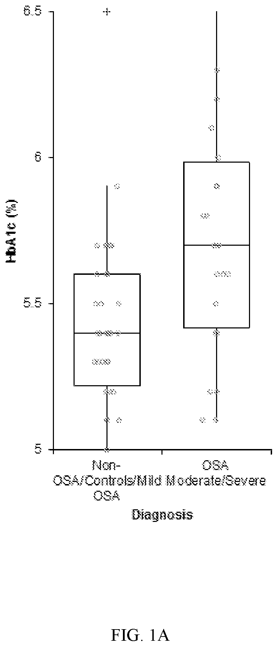

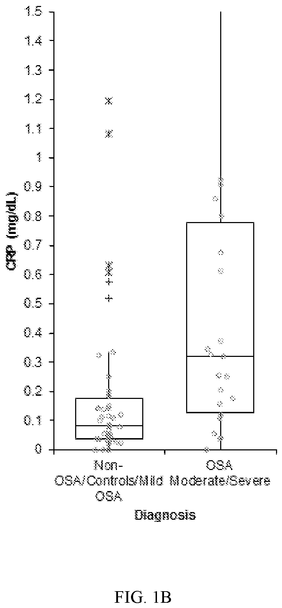

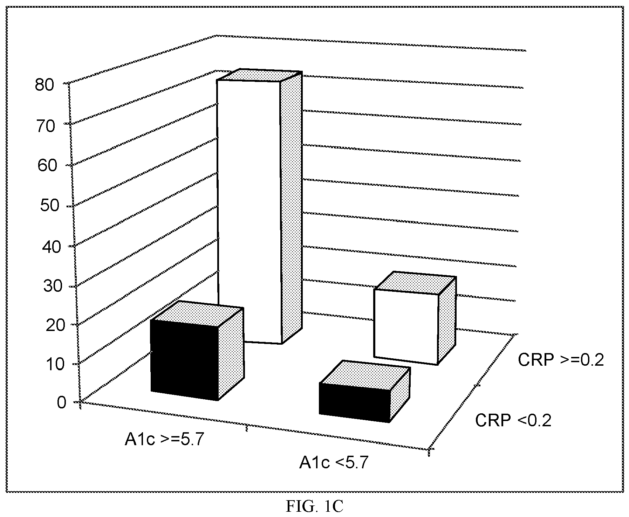

FIGs. 1A and 1B illustrate using HbAlc and CRP, respectively, to distinguish non-OSA/mild OSA subjects from moderate/severe OSA patients. Figure 1C illustrates Probability of moderate/severe OSA by HbAlc and CRP values in combination. This figure shows that HbAlc and CRP are additive for diagnosis of OSA and promising for use in combination for improved diagnostic accuracy.

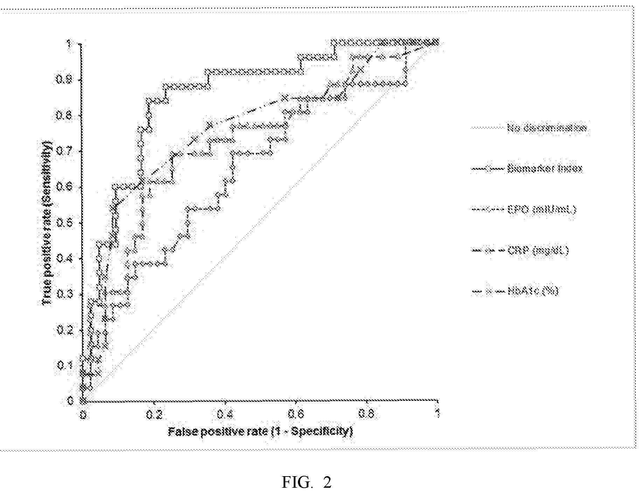

FIG. 2 illustrates ROC curves for detection of moderate/severe OSA. This figure is a comparison of the performance of three biomarkers HbAlc, CRP, EPO used individually with the performance of them and used in combination in a predetermined algorithm for OSA diagnoses. The predetermined algorithm is developed using the "Linear Model - Log Value - 3 Markers" model and is represented by the formula: 12.8117*log(HbA1c) + 0.74983*log(CRP) + 1.53056*log(EPO).

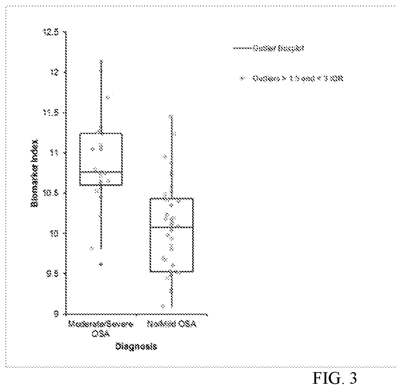

FIG. 3 shows the algorithm, represented by the formula: 12.8117*log(HbA1c) + 0.74983*log(CRP) + 1.53056*log(EPO), can be used to differentiate moderate/severe OSA subjects from non-OSA/mild OSA subjects.

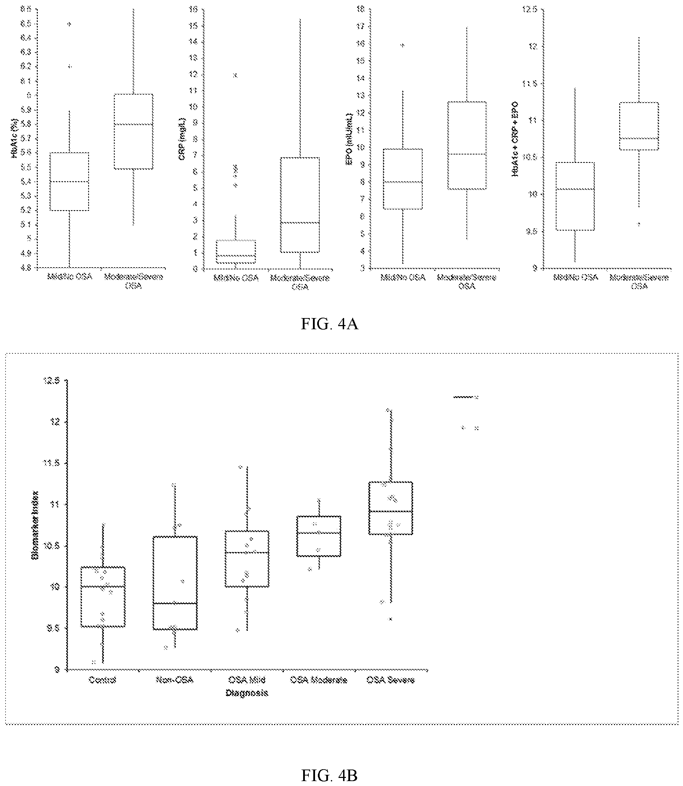

FIG. 4A shows the distribution of biomarkers by diagnostic category (Mid/No OSA vs. Moderate/Severe OSA). FIG. 4B shows the positive association between the multimarker index of the algorithm, represented by the formula: 12.8117*log(HbA1c) + 0.74983*log(CRP) + 1.53056*log(EPO), and the severity of OSA in subjects tested.

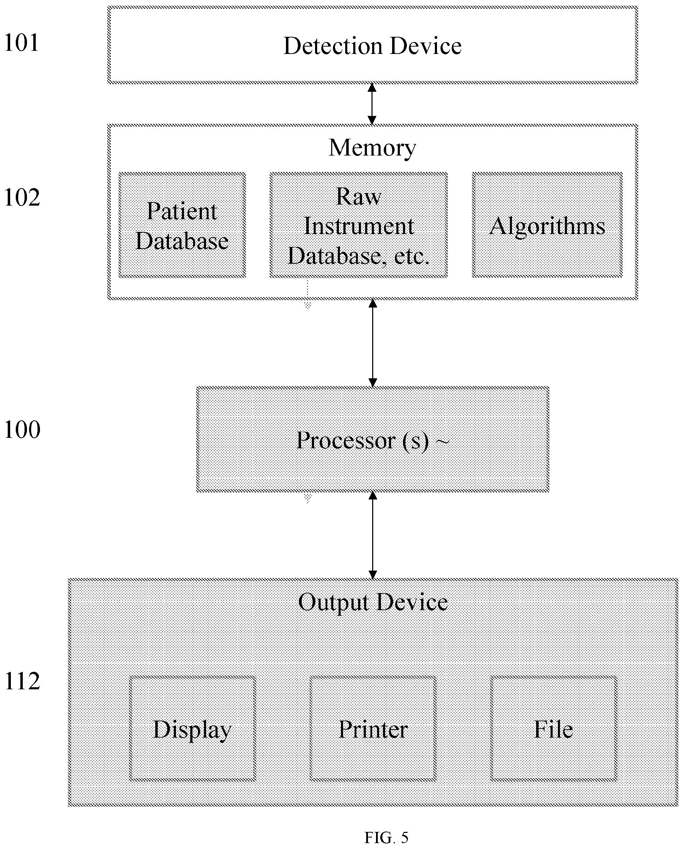

FIG. 5 shows a block diagram of a system that can be used to execute various embodiments of the invention.

### DETAILED DESCRIPTION

In the description that follows, a number of terms are used extensively, the following definitions are provided to facilitate understanding of various aspects of the invention. Use of examples in the specification, including examples of terms, is for illustrative purposes only and is not intended to limit the scope and meaning of the embodiments of the invention herein. Numeric ranges are inclusive of the numbers defining the range, in the specification, the word "comprising" is used as an open-ended term, substantially equivalent to the phrase "including, but not limited to," and the word "comprises" has a corresponding meaning.

The present disclosure provides metabolic and endocrine biomarkers whose expression profiles are related to the assessment, prediction, prognosis, monitoring or diagnosis of OSA in a subject. The invention also provides biomarkers that can be combined into various algorithms to provide accurate diagnosis of OSA in a subject.

### 1. SYMPTOMS OF OBSTRUCTIVE SLEEP APNEA (OSA)

Obstructive sleep apnea (OSA) is a sleep-related breathing disorder that involves a decrease or complete halt in airflow despite an ongoing effort to breathe. It occurs when the muscles relax during sleep, causing soft tissue in the back of the throat to collapse and block the upper airway. This leads to partial reductions (hypopneas) and complete pauses (apneas) in breathing that last at least 10 seconds during sleep. Most pauses last between 10 and 30 seconds, but some may persist for one minute or longer. This can lead to abrupt reductions in blood oxygen saturation, with oxygen levels falling as much as 40 percent or more in severe cases. The brain responds to the lack of oxygen by alerting the body, causing a brief arousal from sleep that restores normal breathing. This pattern can occur hundreds of times in one night. The result is a fragmented quality of sleep that often produces an excessive level of daytime sleepiness.

Most people with OSA snore loudly and frequently, with periods of silence when airflow is reduced or blocked. They then make choking, snorting or gasping sounds when their airway reopens. OSA patients often have excessive daytime sleepiness, and daytime neurobehavioral problems. The other symptoms OSA patients may have include one or more of the following: fluctuating oxygen levels, increased heart rate, chronic elevation in daytime blood pressure, increased risk of stroke, higher rate of death due to heart disease, impaired glucose tolerance and insulin resistance, impaired concentration, mood changes, increased risk of being involved in a deadly motor vehicle accident, and disturbed sleep of the bed partner.

OSA can occur in any age group, but prevalence increases between middle and older age. OSA with resulting daytime sleepiness occurs in at least four percent of men and two percent of women. About 24 percent of men and nine percent of women have the breathing symptoms of OSA with or without daytime sleepiness. About 80 percent to 90 percent of adults with OSA remain undiagnosed. OSA occurs in about two percent of children and is most common at preschool ages.

Certain population are especially at risk of developing OSA, including, people who are overweight (Body Mass Index of 25 to 29.9) and obese (Body Mass Index of 30 and above); men and women with large neck sizes: 17 inches or more for men, 16 inches or more for women; middle-aged and older men, and post-menopausal women; Ethnic minorities; People with abnormalities of the bony and soft tissue structure of the head and neck; Adults and children with Down Syndrome; children with large tonsils and adenoids; anyone who has a family member with OSA; people with endocrine disorders such as Acromegaly and Hypothyroidism; smokers; those suffering from nocturnal nasal congestion due to abnormal morphology, rhinitis or both; people with hypertension, cardiovascular disease and diabetes mellitus, independent of obesity.

Apnea-hypopnea index (AHI) and hypoxemia index are two commonly used standard for assessing the severity of OSA. AHI is an average of the combined number of apneas and hypopneas that occur per hour of sleep; a higher AHI indicates a more severe form of OSA. Non-OSA is indicated by an AHI of less than 5, mild OSA is indicated by an AHI between 5-14.9, moderate OSA is indicated by an AHI between 15 and 29.9, and severe OSA is indicated by an AHI_greater or equal than_30. Hypoxemia index is measured by the percent sleep time with oxyhemoglobin saturation < 90%. A higher Hypoxemia index indicates a more severe form of OSA than a lower Hypoxemia index. Generally, normal individual or slight OSA has an Hypoxemia index lower than < 0.5, mild OSA has a Hypoxemia index between 0.5-4.9, moderate OSA has a Hypoxemia index between 5 and 9.9, and severe OSA has a Hypoxemia index greater than or equal to 10%.

There are several therapies for OSA patients. Continuous positive airway pressure (CPAP) is the standard therapy for moderate to severe cases of OSA and a good option for mild OSA. CPAP provides a steady stream of pressurized air to patients through a mask that they wear during sleep. This airflow keeps the airway open, preventing pauses in breathing and restoring normal oxygen levels. Newer CPAP models are small, light and virtually silent. Patients can choose from numerous mask sizes and styles to achieve a good fit. Heated humidifiers that connect to CPAP units also contribute to patient comfort.

An oral appliance is also an effective treatment option for people with mild to moderate OSA who either prefer it to CPAP or are unable to successfully comply with CPAP therapy. Oral appliances look much like sports mouth guards, and they help maintain an open and unobstructed airway by repositioning or stabilizing the lower jaw, tongue, soft palate or uvula. Some are designed specifically for snoring, and others are intended to treat both snoring and OSA. They should always be fitted by dentists who are trained in sleep medicine.

Surgery is a treatment option for OSA when noninvasive treatments such as CPAP or oral appliances have been unsuccessful. It is most effective when there is an obvious anatomic deformity that can be corrected to alleviate the breathing problem.

Weight loss may also benefit some people with OSA, and changing from back-sleeping to side-sleeping may help those with mild cases of OSA.

### 2. DIAGNOSING OSA WITH BIOMARKERS

The present disclosure provides biomarkers, as listed in Table 1, related to the assessment, prediction, monitoring or diagnosis of OSA in a subject. Many of the biomarkers are involved in a variety of biological processes, such as stress, inflammation, and visceral obesity.

**Table 1. Biomarkers related to OSA.**

| Biomarker | Description | Expected results in OSA patients |
|---|---|---|
| HbA1c | Hemoglobin A1c | High |
| CRP | C-reactive protein | High |
| IL-6 | Interleukin-6 | High |
| Uric acid | Uric acid | High |
| EPO | Erythropoietin | High |

Hemoglobin A1c (HbAlc) refers to glycosylated hemoglobin. HbAlc is associated with diabetes. Subjects without diabetes have HbAlc in the level range of 20-41 mmol/mol (4-5.9%); HbAlc levels between 5.7% and 6.4% indicate increased risk of diabetes. HbAlc is also associated with OSA. Our studies have shown that a HbAlc level equal or higher than 5.7% indicates an individual is at a high risk of OSA.

CRP: C-reactive protein (CRP) is a member of the pentraxin protein family and is an important marker of endothelial dysfunction in the pathogenesis of coronary artery disease. It is a product and mediator of low-grade inflammation that occurs in atherosclerosis and is often found in the atherosclerotic plaque. Increased CRP levels have been shown to be an independent risk predictor for peripheral vascular disease, myocardial infarction, stroke, and vascular death. See, Guven et al., Sleep Breath (2012) 16:217-221. However, the relationship between CRP and OSA was inconclusive. See Motesi et al., CHEST (2012) 142 (1) 239-245. Our studies have shown that a level above 0.2 mg/dL in serum indicates an individual is at a high risk of OSA.

IL-6 is a cytokine that functions in inflammation and the maturation of B cells. It is primarily produced at sites of acute and chronic inflammation, where it is secreted into the serum and induces a transcriptional inflammatory response through interleukin 6 receptor, alpha. The functioning of this protein is implicated in a wide variety of inflammation-associated disease states, including diabetes mellitus and systemic juvenile rheumatoid arthritis. Its levels are elevated in patient with cardiovascular diseases and/or OSA. See Chami et al., SLEEP, (2013) Vol. 36, No. 5 763-768; Maeder et al., Clin Biochem. (2015) Mar. 48(4-5):340-346. The severity of OSA is positively correlated with the level of IL-6.

Uric acid is formed as a result of the activity of xanthine oxidase, an enzyme that plays a mechanistic role in oxidative stress and cardiovascular diseases. OSA patients have elevated uric acid levels. OSA patients suffer from repeated upper airway obstruction episodes, causing an intermittent state of hypercapnia and hypoxia. This is accompanied by decreased blood oxygen saturation and arousals during sleep. Inadequate oxygen supplies can impair the formation of adenosine triphosphate (ATP), an important compound for cellular homeostasis. This leads to a net degradation of ATP to adenosine diphosphate and adenosine monophosphate. Thus, this process causes the release of purine intermediates (adenosine, inosine, hypoxanthine and xanthine), ending with an overproduction of uric acid, the purine final catabolic product. The production of uric acid is often accompanied by the enhanced synthesis of reactive oxygen species (ROS), which can cause hypoxia-related tissue damage. See, Hirotsu, et al., PLOS ONE, June 2013 Vol. 8 (6) 1-9.

Erythropoietin (EPO) is the principal hormone involved in the regulation of erythrocyte differentiation and the maintenance of a physiological level of circulating erythrocyte mass. EPO is a member of the EPO/TPO family and is a secreted, glycosylated cytokine composed of four alpha helical bundles. The protein is found in the plasma and regulates red cell production by promoting erythroid differentiation and initiating hemoglobin synthesis. This protein also has neuroprotective activity against a variety of potential brain injuries and antiapoptotic functions in several tissue types. Increased EPO is associated with cardiovascular abnormalities independent of blood pressure level. OSA patients have increased serum EPO levels due to patients' kidneys' reacting to hypoxia by enhancing the production of circulating erythropoietin.

### 3. DETERMINATION OF COMBINATIONS OF BIOMARKERS AND ALGORITHMS FOR THE DIANGOSIS.

### A. Identifying OSA-related biomarkers.

This invention provides biomarkers that can be used in combinations for OSA diagnoses. The biomarkers include those listed in Table 1. A biomarker can be used if its level in a subject, relative to a reference value, correlates with the status of OSA. A higher or lower than that reference value correlates with the presence of OSA.

A reference value for a biomarker can be a number or value derived from population studies, including without limitation, such subject having the same or similar age range, subjects in the same or similar ethnic group. Such reference values can be obtained from mathematical algorithms and computed indices of OSA which are derived from statistical analyses and/or risk prediction data of populations, for example, a population with OSA, a population without OSA or having only mild OSA, a population cured from OSA. A reference value can be derived from ROC curve analysis, selecting the reference value as that which maximizes sensitivity while keeping the specificity above a user-defined threshold, or as that which maximizes specificity while keeping the sensitivity above a user-defined threshold. A user-defined threshold may be 80% sensitivity.

The reference value may be the amount (i.e. level) of a biomarker in a control sample derived from one or more subjects who do not have OSA (i.e., healthy, and/or non-OSA individuals).Retrospective measurement of biomarkers in properly banked historical subject samples may be used in establishing these reference values, thus shortening the study time required. Alternatively, the reference value can be derived from a database of biomarker patterns from previously tested subjects.

The reference may be determined based on the range of the levels of the biomarker in the healthy subjects. If the biomarker is one that is increased in OSA patients, e.g., HbAlc, the reference value can be, e.g., the upper limit of the range of levels of the biomarker in subjects who do not have OSA. If the biomarker is one that is decreased in OSA patients, the reference value for that biomarker can be the lower limit of the range of the levels of the biomarker in subjects that do not have OSA.

Some biomarkers are shown to have additive effects in determining OSA status. For example, while patients having OSA in general have high HbAlc level (≥ 5.7%) or high CRP level (≥0.2), the percentage of patients having OSA in the group having both high HbAlc (≥ 5.7%) and high CRP (≥0.2) is 73%, much higher than the percentage of patients having OSA in the group having high level of either only HbAlc or only CRP, 19% and 19%, respectively. See Figure 1C. This suggests HbAlc and CRP can be used in combination in OSA diagnosis.

### B. Determining biomarker combinations and algorithms

Various biomarkers are chosen based on their performance in differentiating subjects having OSA from those having no OSA. The performance of each biomarker can be evaluated by the determination of Areas Under the Curve (AUC) of Receiver Operating Characteristic (ROC) curves, See Table 4. Individual biomarkers having an AUC equal to or greater than 0.6, for example, HbAlc or CRP, can be used in various combinations and evaluated for their performance in OSA diagnosis, as described below.

The present invention provides OSA diagnostic methods using algorithms of various combinations of the biomarkers listed in Table 1. Many of these biomarkers play important roles in one or more physiological or biological pathways, e.g., metabolic pathways or endocrine pathways. Combinations of some biomarkers provide performance characteristics of the diagnosis that is superior to that of the individual biomarkers. This is shown by the results in Table 6 and Figure 2, which indicate diagnostic tests using various combinations of biomarkers yielded better AUCs, sensitivities, and specificities than their respective individual biomarker components - when proper mathematical and clinical algorithms are used.

Mathematical algorithms useful for OSA diagnosis, such as the ones used in the experiments giving rise to the results of Table 5, can be generated from a defined dataset using statistical analysis that is known in the art. The dataset include serum levels of the biomarkers and clinical characteristics of the subjects in the study. The subjects are classified as having moderate/severe OSA and subjects having mild/no OSA based on a number of standard clinical parameters from sleep study results, for example, their AHI measurements: mild OSA (5-14.9), moderate OSA(15-29.9), or severe OSA (≥30). Mean and minimum oxygen saturation - the two additional measures of OSA severity - were also measured and used in the classification: healthy (around 95 percent), mild to moderate OSA (80 to 85 percent) is moderate, severe OSA (79 percent or less). See, http://www.sleepapnea.org/treat/diagnosis/sleep-study-details.html.

Pearson (r) and Spearman (p) correlation coefficients can be used, for Gaussian and skewed variables, to determine the correlation between one of the biomarkers and one of clinical parameters, e.g., AHI, Minimum Oxygen saturation (Min O₂), BMI and ESS. Two-tailed descriptive statistics for each variable were performed using Student's t-test. ANOVA, or Wilcoxon test for continuous variables depending upon data distribution and normality, and Fisher's Exact test or Chi-square test were used for dichotomous variables. The statistical correlations between various biomarkers and the clinical parameters are shown in Table 3.

Various classification models can be then applied to the dataset comprising the combinations of biomarkers, which have been shown to have a correlation with the clinical characteristics of OSA. These models are well known in the art, including, but are not limited to, Linear Model, Non-Linear Model, Linear DA, quadratic DA, Naive Bayes, Linear Regression, Quadratic Regression, KNN, Linear SVM, SVM with 2^{nd} order polynomial Kernel, SVM with 3^{rd} order polynomial Kernel, Neural Networks, Parzen Windows, Fuzzy Logic, and Decision Trees. A plurality of algorithms combining various biomarkers is therefore produced. For example, applying the above classification models to combinations of biomarkers - selected from those listed in Table 3 - and the Body-Mass Index (BMI) produced a set of algorithms. A subset of these algorithms is shown in Table 5. Each algorithm takes in the levels of the various biomarkers and produces a multimarker index for these biomarkers for each sample tested. In some approaches, expression levels of biomarkers are processed into more valuable forms of information prior to their presentation to the algorithm, e.g., by using either common mathematical transformations such as logarithmic or logistic functions. Other data processing approaches, such as normalization of biomarker results in reference to a population's mean values, etc. are also well known to those skilled in the art and can be used.

The algorithms produced are in the forms of mathematical functions combining values of the levels of biomarkers. As used in this disclosure, an algorithm of a "Linear Model - Linear Value" combination is generated using the linear model and uses the original values of the levels of biomarkers in calculating the multimarker index; an algorithm of a "Linear Model - Log Value" combination is generated using the linear model and uses logarithmic values of the levels of biomarkers in calculating the multimarker index; an algorithm of a "Non-linear Model - Linear Value" combination is generated using the Non-linear Model and uses the original values of the levels of biomarkers in calculating the multimarker index; an algorithm of a "Non-linear Model - Log Value" combination is generated using the Non-linear Model and uses logarithmic values of the levels of biomarkers in calculating the multimarker index. If a model is non-linear, cross terms, in addition to biomarker itself, will be used in the multimarker index. See Table 5 and the notes below for the description of some exemplary algorithms.

Although various algorithms are described here, several other model and formula types beyond those mentioned herein are well known to one skilled in the art and can also be used to generate algorithms useful for the diagnosis, for example, as disclosed in US 2011/0137851 A1.

Each of the various algorithms produced is evaluated for its suitability as a diagnosis method for OSA, based on standard performance metrics, such as Areas Under the Curve (AUC), and all corresponding combinations of diagnostic sensitivity and specificity. The algorithms used in the invention for OSA diagnosis have an AUC greater than 0.7. The AUC may be greater than or equal to 0.75, greater than or equal to 0.80, greater than or equal to 0.81, greater than or equal to 0.82, greater than or equal to 0.83, greater than or equal to 0.84, greater than or equal to 0.85, greater than or equal to 0.86, greater than or equal to 0.87, greater than or equal to 0.88, greater than or equal to 0.89. The algorithms used also have specificity and sensitivity that is suitable for the OSA diagnosis. The diagnosis using combinations of biomarkers disclosed herein may have a specificity of at least 60%, at least 66%, at least 72%, at least 77%, at least 79%, at least 81%, at least 83%, at least 85%, at least 92%, at least 94% -- when the sensitivity of the assay is fixed at 80%; and has a specificity of at least 51%, 55%, 60%, 62%, 75%, 77%, 79%, 85%, or 89% - when the sensitivity of the assay is fixed at 85%.

Other factors can also be considered in selecting algorithms of combinations of biomarkers for the diagnosis, e.g., whether the algorithm can provide a means for assessing disease burden and severity and for measuring response to treatment; and whether the biomarkers used in the algorithm are on a causal pathway known to relate to development of OSA. Tests having these useful features could obviate the need screening questionnaires, and possibly for polysomnography, at least for some patients, and can be used to track response to a therapy of OSA.

According to the invention, the biomarkers used in the methods are HbAlc and one or more of the biomarkers selected from the group consisting of CRP, IL-6, uric acid, and EPO (Table 1).

Methods may rely upon a combination of measured levels of HbAlc and CRP. Algorithms may be used, such as Linear Model-Linear Value, Linear Model-Log Value, Non-linear Model-Linear Value, or Non-linear Model-Log Value combination. The algorithm may be a Non-linear Model-Linear Value combination of HbAlc and IL-6.

The algorithm may be a combination of HbAlc, CRP and EPO, and the combination is Linear Model - Linear Value, Linear Model - Log Value, or Non-linear Model - Linear Value combination.The algorithm may be a Linear Model-Linear Value combination of HbAlc, uric acid and EPO. The algorithm may be a Non-linear Model-Linear Value combination of HbAlc, CRP and IL-6. The algorithm may be a Linear Model-Linear Value combination of HbAlc, CRP, and uric acid. The algorithm may be a Non-linear Model-Linear Value combination of HbAlc, IL-6, and EPO.

### 4. SAMPLE COLLECTION

Various sample types can be used for analyzing the biomarker expression in a subject, including, but are not limited to, whole blood, serum, plasma, saliva, mucus, breath, urine, CSF, sputum, sweat, stool, hair, seminal fluid, biopsy, rhinorrhea, tissue biopsy, cytological sample, platelets, reticulocytes, leukocytes, epithelial cells, or whole blood cells. A tissue or organ sample, such as a non-liquid tissue sample maybe digested, extracted or otherwise rendered to a liquid form - examples of such tissues or organs include cultured cells, blood cells, skin, liver, heart, kidney, pancreas, islets of Langerhans, bone marrow, blood, blood vessels, heart valve, lung, intestine, bowel, spleen, bladder, penis, face, hand, bone, muscle, fat, cornea or the like. One or more samples may be collected from a subject at any time, including before a diagnosis of OSA, before a treatment for OSA, during the course of the treatment, and at any time following the treatment.

In some embodiments, the sample is a blood sample. The blood samples from patients and controls may be collected and processed prior to a diagnosis or initiation of any treatment. Whole blood samples can be shipped at 4 °C for immediate HbAlc testing or stored at 4 °C up to 7 days before being tested. Frozen whole blood samples can be stored at - 20° up to 3 months and at -70°C up to 18 months for HbAlc testing. Plasma or serum samples can be dispensed into cryo-tubes and stored at -70 °C for a period of time before being tested for CRP, IL-6, uric acid, or EPO.

### 5. TESTING AND MEASURING THE MARKERS IN SERUM

Various well-known immunological methods can be used to specifically identify and/or quantify the disclosed biomarkers, such as EPO and IL-6. These methods include, but are not limited to, immunologic- or antibody-based techniques include enzyme-linked immunosorbent assay (ELISA), radioimmunoassay (RIA), western blotting, immunofluorescence, microarrays, some chromatographic techniques (i.e. immunoaffinity chromatography), flow cytometry, immunoprecipitation. These methods are based on the specificity of an antibody or antibodies for a particular epitope or combination of epitopes associated with the analyte, protein or protein complex of interest.

Methods of producing antibodies for use in protein or antibody arrays, or other immunology based assays for detection of the biomarkers disclosed herein are known in the art. For preparation of monoclonal antibodies directed towards a biomarker, any technique that provides for the production of antibody molecules by continuous cell lines in culture may be used. Such techniques include, but are not limited to, the hybridoma technique originally developed by Kohler and Milstein Nature (1975) 256:495-497, the trioma technique (Gustafsson et al., Hum. Antibodies Hybridomas (1991) 2:26-32), the human B-cell hybridoma technique (Kozbor et al., Immunology Today (1983) 4:72), and the EBV hybridoma technique to produce human monoclonal antibodies (Cole et al., In: Monoclonal Antibodies and Cancer Therapy, Alan R. Liss, Inc., (1985) 77-96). In addition, techniques described for the production of single chain antibodies (U.S. Patent 4,946,778) can be adapted to produce a biomarker -specific antibodies. An additional embodiment of the invention utilizes the techniques described for the construction of Fab expression libraries (Huse et al., Science (1989) 246:1275-1281) to allow rapid and easy identification of monoclonal Fab fragments with the desired specificity for a biomarker proteins. Non-human antibodies can be "humanized" by known methods (e.g., U.S. Patent No. 5,225,539).

Antibody fragments that contain the idiotypes of a biomarker can be generated by techniques known in the art. For example, such fragments include, but are not limited to, the F(ab')2 fragment which can be produced by pepsin digestion of the antibody molecule; the Fab' fragment that can be generated by reducing the disulfide bridges of the F(ab')2 fragment; the Fab fragment that can be generated by treating the antibody molecular with papain and a reducing agent; and Fv fragments. Synthetic antibodies, e.g., antibodies produced by chemical synthesis, are useful in the present invention.

Serum or plasma EPO may be measured by a chemiluminescent immunoassay. A sample is added to a reaction vessel along with the paramagnetic particles coated with mouse monoclonal anti-EPO, blocking reagent and the alkaline phosphatase conjugate. After incubation in a reaction vessel, materials bound to the solid phase are held in a magnetic field while unbound materials are washed away. Then, the chemiluminescent substrate is added to the vessel and light generated by the reaction is measured with a luminometer. The light production is directly proportional to the level of EPO in the sample. The amount of analyte in the sample is determined from a stored, multi-point calibration curve. Various kits and protocols are commercially available for testing of EPO, e.g., BCI Item No. A16364, which can be used in conjunction with BCI's Access Immunoassay systems.

Serum or plasma IL-6 may be measured using an immunoenzymatic assay, *e.g.,* a one-step immunoenzymatic ("sandwich") assay. A sample is added to a reaction vessel along with the paramagnetic particles coated with mouse monoclonal anti-human IL-6, blocking reagent and the alkaline phosphatase conjugate. After incubation in a reaction vessel, materials bound to the solid phase are held in a magnetic field while unbound materials are washed away. Then, a chemiluminescent substrate is added to the vessel and light generated by the reaction is measured with a luminometer. The light production is directly proportional to the level of IL-6 in the sample. The amount of IL-6 in the sample is determined from a stored, multi-point calibration curve.

HbAlc level can be measured by a turbidimetric immunoinhibition method and is typically expressed as a percentage of the total hemoglobin in the blood sample. A system using two unique cartridges, Hb and A1c may be employed and a hemoglobin reagent is used in a colorimetric reaction with the whole blood sample. The system automatically proportions the appropriate sample and reagent volumes into the reaction cuvette, typically at a ratio of one part sample to 8.6 parts reagent. The change in absorbance at 410 nanometers, which is directly proportional to the concentration of total hemoglobin in the sample, is monitored and used to calculate and express total hemoglobin concentration. The HbAlc level is determined by a reaction in which hemoglobin A1c antibodies combine with hemoglobin A1c from the sample to form soluble antigen-antibody complexes. Polyhaptens from the reagent then bind with the excess antibodies and the resulting agglutinated complex is measured turbidimetrically, i.e., by monitoring the change in absorbance at 340 nanometers. This change in absorbance is inversely proportional to the level of HbAlc in the sample and can be used to calculate HbAlc level. The HbAlc level is typically expressed as a percentage of total hemoglobin according to the formula: % HbAlc = (A1c (g/dL)/ Hb (g/dL)) x 100.

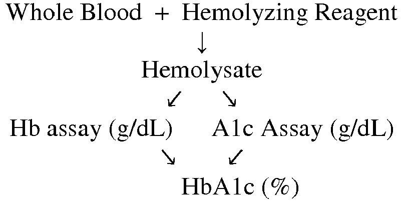

One exemplar assay for HbAlc levels involves the use of four reagents: a Total Hemoglobin reagent, a HbAlc R1 antibody reagent, a HbAlc R2 agglutinator reagent and a Hemoglobin Denaturant. The assay is conducted as follows: in a pretreatment step, the whole blood is mixed with Hemoglobin Denaturant in a 1:41 dilution and incubated for a minimum of five minutes at room temperature. The red blood cells are lysed and the hemoglobin chain is hydrolyzed by protease present in the reagent. Total hemoglobin is measured via the conversion of all hemoglobin derivatives into alkaline hematin in the alkaline solution of a non-ionic detergent. Addition of the pre-treated blood sample to the Total Hemoglobin reagent results in a green solution, which is measured at 600nm. HbAlc is measured in a latex agglutination assay. An agglutination, consisting of a synthetic polymer containing multiple copies of the immunoreactive portion of HbAlc, causes agglutination of latex coated with HbAlc specific mouse monoclonal antibodies. In the absence of HbAlc in the sample, the antibody-coated microparticles in the HbAlc R1 and the agglutinator in the HbAlc R2 will agglutinate. Agglutination leads to an increase in the absorbance of the suspension. The presence of HbAlc in the sample results in a decrease in the rate of agglutination of the HbAlc R1 and the agglutinator in the HbAlc reagent R2. The increase in the absorbance, is therefore , inversely proportional to the concentration of HbAlc in the sample. The increase in the absorbance is measured at 700 nm.

Methods of measuring CRP level typically employs a turbidimeter to measure the reduction of incidence light due to reflection, absorption, or scatter of immune complexes formed in solution between CRP of the patient serum and anti-CRP antibodies. The anti-CRP antibodies may be rabbit anti-CRP antibodies. The anti-CRP antibodies can be introduced in various ways, for example, via latex particles on which the anti-CRP antibodies are coated. The anti-CRP antibody-coated particles bind to CRP in the sample, resulting in the formation of insoluble aggregates causing turbidity, which can be monitored by detecting the change in absorbance at 940 nm. The amount of insoluble aggregates formed is proportional to the level of C-reactive protein in the sample. In one exemplar assay, the volume ratio of the sample to anti-CRP antibody reagent is 1:26. The CRP levels can then be determined based on the change in absorbance at 940 nm and a pre-determined calibration curve.

Non-immunological methods, include those based on the physical or chemical properties of the biomarkers, can be also be used to measure the disclosed biomarkers. Numerous methods are well known in the art and can be used to analyze/detect products of various reactions involving a biomarker of the invention. The reaction products can be detected by means of fluorescence, luminescence, mass measurement, or electrophoresis, etc. Furthermore, reactions can occur in solution or on a solid support such as a glass slide, a chip, a bead, or the like.

Uric acid is typically measured using non-immunological methods. For example, one method of measuring uric acid is based on that uric acid can be oxidized by uricase to produce allantoin and hydrogen peroxide. Using this method, the hydrogen peroxide so produced reacts with 4-aminoantipyrine (4-aap) and 3,5-dichloro-2-hydroxybenzene sulfonate (dchbs) in a reaction catalyzed by peroxidase to produce a colored product. A change in absorbance at 520 nanometers is monitored. This change is directly proportional to the level of uric acid in the sample and is used to calculate and determine the uric acid level.

In another exemplar assay of measuring uric acid, also based on that uric acid can be converted by uricase to allantoin and hydrogen peroxide. The hydrogen peroxide reacts with N,N-bis(4-sulfobutyl)-3,5-dimethylaniline, disodium salt (MADB) and 4-aminophenazobe in the presence of peroxidase to produce a chromophore, which is then read biochromatically at 660/800 nm. The amount of dye formed is proportional to the uric acid concentration in the sample and thus can be used to determine the level of uric acid.

Commercial kits and devices are readily available to measure any of the aforementioned biomarkers.

### 6. DIAGNOSIS OF OSA USING A MULTIMARKER INDEX PRODUCED FROM A PREDETERMINED ALGORITHM.

Diagnosis of OSA may be made by calculating a multimarker index based on the combinations of two or more biomarkers in a subject using a predetermined algorithm as described above. The biomarkers used in the algorithm in a subject are measured and the values are fed to the algorithm to produce a multimarker index. The multimarker index of the subject is then compared with a reference value to determine if the subject has OSA.

The reference value for the multimarker index of a particular algorithm may be determined by ROC analysis, comparing a population with No/Mild OSA versus a population with Moderate/Severe OSA. A reference value can be derived from ROC analysis, selecting the reference value as that which maximizes sensitivity while keeping the specificity above a user-defined threshold. The reference value can also be selected as that which maximizes specificity while keeping the sensitivity above a user-defined threshold. The reference value may be selected such that the specificity is at the maximum when the user-defined threshold of sensitivity is 80% based on the ROC analysis.

The reference value may be determined based on the range of the multimarker indices in the healthy subjects. If the multimarker index is one that is increased in OSA patients, the reference value can be, e.g., the upper limit of the range of the multimarker indices in subjects do not have OSA; and the subject is diagnosed as having OSA if his or her multimarker index is higher than the reference value. If the multimarker index is one that is decreased in OSA patients, the reference value can be the lower limit of the range of the multimarker index in subjects do not have OSA; and the subject is diagnosed as having OSA if his or her multimarker index is lower than the reference value.

The invention may also be useful in determining the severity of OSA. If the multimarker index is one that is increased in OSA patients, a higher multimarker index indicates a more severe form of OSA, and vice versa. See Figure 4B. This information is useful in determining the type of treatment each patient should receive. For example, a subject having a severe form of OSA may require immediate Continuous positive airway pressure (CPAP) or even surgery; and a subject having a mild form of OSA may be advised to have a positive life style change, for example, weight loss. In addition, the information may also be used to prioritize treatment; a patient having a higher multimarker index may require attention and treatment sooner than a patient having a lower multimarker index.

### 7. CLINICAL VALIDATION OF THE DIAGNOSIS

A subject who has been diagnosed with OSA using the biomarkers or combinations thereof may also be evaluated for one or more clinical characteristics of OSA, which include, questionnaires with or without medical history and physical examination, audiotaping, videotaping, pulse oximetry, polysomnography, abbreviated polysomnography (aPSG), and home-based polygraphy. Measurements in one or more of these characteristics that are consistent with the known symptoms for OSA patients would confirm the diagnosis.

In some embodiments, prior to being diagnosed with OSA using the biomarkers approach, the subject's BMI, Diastolic blood pressure, Systolic blood pressure, and Epworth Sleepiness Scale are measured. Body mass index (BMI) is a person's weight in kilograms divided by height in meters squared. Normal BMI is 18-24.9; overweight is 25.0-29.9; and obese is greater than 30. A BMI greater than 40 is morbidly obese. Diastolic blood pressure and systolic blood pressure are also known to increase with patients having OSA. The Epworth Sleepiness Scale is a subjective measure of a patient's sleepiness. The test is a list of eight situations in which a patient rates his or her tendency to become sleepy on a scale of 0, no chance of dozing, to 3, high chance of dozing. The eight situations are: sitting and reading, watching TV, sitting inactive in a public place, as a passenger in a car for an hour without a break, lying down to rest in the afternoon when circumstances permit, sitting and talking to someone, sitting quietly after a lunch without alcohol, in a car while stopped for a few minutes in traffic. The values of the patient's responses to the situations are added up to produce a total score based on a scale of 0 to 24. The scale estimates whether a patient is experiencing excessive sleepiness that possibly requires medical attention. A value between 0-9 means the patient has an average amount of daytime sleepiness. A value between 10-15 means the patient is excessively sleepy depending on the situation and may need to consider seeking medical attention. A value between 16-24 means the patient is excessively sleepy and should seek medical attention. Thus, BMI value, Diastolic blood pressure, or Systolic blood pressure, or ESS that is higher than normal in the subject would increase the level of suspicion that a subject has OSA.

The subject being diagnosed with OSA using the biomarkers approach may also undergo a standard, overnight in-laboratory polysomnographic evaluation. See, American Academy of Sleep Medicine (AASM), International classification of Sleep Disorders. Westchester, IL: AASM; 2005. An apnea hypopnea index (AHI) greater than 5 or a blood oxygen level that is less than 90% in the subject would confirm a diagnosis of OSA. An AHI greater than 15 would confirm that the subject has moderate to severe OSA.

### 8. EVALUATING EFFICACY OF AN OSA THERAPY

The present invention also provides methods to determine whether a therapy is effective for treating OSA. In one embodiment, the method comprises determining the expression levels of the biomarkers before and after the therapy, determining the therapy is effective if the levels of the biomarkers are decreased compared to the levels of the biomarkers before the treatment.

In another embodiment, the method of determining whether the therapy is effective comprises measuring the levels of the biomarkers in a sample from the subject after the therapy; comparing the measured levels of the biomarkers against a predetermined reference value; and determining the therapy is effective if the levels of the biomarkers are lower than the predetermined reference value.

In some embodiments, after the initial determination of the effectiveness of a therapy using the biomarkers, clinical characteristics of OSA are assessed, as described above, to confirm that the therapy is effective.

### 9. KITS

Also described are kits for use in diagnosing OSA. The kit may comprise reagents for specific and quantitative detection of one, two, three or more of the biomarkers in Table 1, along with instructions for the use of such reagents and methods for analyzing the resulting data. For example, the kit may comprise antibodies or fragments thereof, specific for the proteomic markers (primary antibodies), along with one or more secondary antibodies that may incorporate a detectable label; such antibodies may be used in an assay such as an ELISA. Alternately, the antibodies or fragments thereof may be fixed to a solid surface, e.g. an antibody array. The kit may contain a detectable label such as fluorescein, green fluorescent protein, rhodamine, cyanine dyes, Alexa dyes, luciferase, radiolabels, among others. The kit may be used alone for predicting or diagnosing a subject's OSA, or it may be used in conjunction with other methods for determining clinical variables, polysomnography, or other assays that may be deemed appropriate. Instructions or other information useful to combine the kit results with other methods, *e.g*., clinical characteristics studies, to provide a OSA diagnosis may also be provided.

### 4.COMPUTER READABLE MEDIUM AND SYSTEMS

This invention also provides a non-transitory computer readable medium having computer-executable instructions, which when executed, causes a processor to perform a method of the invention. The two or more biomarkers can also be a combination of two or three biomarkers selected from the combinations listed in Table 5. In preferred embodiments, the biomarkers used for the multimarker index determination comprise HbAlc and CRP. In some embodiments, the biomarkers comprise at least one of EPO, IL-6, or uric acid in addition to HbAlc and CRP.
The data that the process accesses may also include additional parameters attributed to the subject, such as BMI and age, which can be used to assist the diagnosis. The processor, executing the instructions embodied in the computer readable medium, also executes a predetermined algorithm to produce a multimarker index of the two or more biomarkers. The predetermined algorithm is selected using the method described above, see the section entitled "DETERMINATION OF COMBINATIONS OF BIOMARKERS AND ALGORITHMS FOR THE DIANGOSIS." The patient can be diagnosed as having OSA if the multimarker index from the sample is higher than a predetermined reference value for that multimarker index and the predetermined algorithm is positive logic. The patient can also be diagnosed as having OSA if the multimarker index is lower than a predetermined reference value for that multimarker index and if the predetermined algorithm is negative logic.

The non-transitory computer readable medium may be, but is not limited to, an electronic, magnetic, optical, electromagnetic, infrared, or semiconductor system, apparatus, device, or propagation medium. More specific examples (a non-exhaustive list) of the computer-readable medium would include the following: an electrical connection having one or more wires, a portable computer diskette, a random access memory (RAM), a read-only memory (ROM), an erasable programmable read-only memory (EPROM or Flash memory), an optical fiber, and a portable compact disc read-only memory (CD-ROM). Note that the non-transitory computer-readable medium could even be paper or another suitable medium, upon which the program is printed, as the program can be electronically captured, via, for instance, optical scanning of the paper or other medium, then compiled, interpreted or otherwise processed in a suitable manner if necessary, and then stored in a computer memory.

This invention also provides a system for diagnosing OSA. FIG. 5 is a block diagram of a computer system that can be used to perform the methods of the invention. The system comprises a detection device **101** configured to measure two or more biomarkers selected from the group consisting of HbAlc, CRP, IL-6, uric acid, and EPO in a subject or any of the combinations of biomarkers described above.

The system further comprises an analyzing device that is in communication with the detection device, the analyzing device comprising a variety of typical computer components, including a non-transitory computer readable medium, *e.g*., a memory **102,** and one or more computer processors **100.** The analyzing device may also comprise a database storing predetermined algorithms and reference values for each of the multimarker indices produced by the algorithms. As stated above, the non-transitory computer readable medium also hosts computer-executable instructions, when executed, causes a computer processor to access data attributed to a sample from a patient, e.g., from patient databases or raw instrument databases associated with the detection device; to execute a predetermined algorithm to compute a multimarker index; and to compare the multimarker index with the reference values to determine the status of OSA of the patient.

The system can optionally comprise an output device **112,** such as a display, a printer, or a file, to output the result of the diagnosis. The output device may be a display, *e.g.,* a monitor, which can display a signal indicating that a patient has OSA if the sample from the subject has a multimarker index higher than the predetermined reference value and if the predetermined algorithm is positive logic; or displays a signal indicating that a subject has OSA if the sample from the subject has a multimarker index lower than the predetermined reference value and if the predetermined algorithm is negative logic.

This invention thus also provides a computer implemented method for diagnosing OSA. The method may comprise determining the levels of two or more biomarkers in a sample from a patient, determining a multimarker index for the two or more biomarkers using a predetermined algorithm with a computer processor; comparing the multimarker index with a predetermined reference value for the multimarker index; and diagnosing the patient as having OSA if the multimarker index is higher than the predetermined reference value and the predetermine algorithm is positive logic; or diagnosing the patient as having OSA if the multimarker index is lower than the predetermined reference value and the predetermined algorithm is negative logic. The method step of comparing the multimarker index with a predetermined reference value, or the step of diagnosing, or both methods steps, may be conducted with one or more computer processors.

Processors executing the any of the above algorithms can be programmed into the analyzing device in a number of ways.
(1) The UDR (User Defined Reagent) option is where a user, e.g., a device manufacturer engineer, a physician or laboratorian, first prescribes a test of a combination of the biomarkers in this disclosure, particularly those selected from the group consisting of HbAlc, CRP, IL-6, uric acid, and EPO. Device manufacturer or any lab/clinical facility with or without assistance from the device manufacturer, will then choose a suitable algorithm having the prescribed combination, and program the algorithm into the device. This option gives users the flexibility to choose an algorithm that most suits their needs.
(2) The Database Kit option is where the device manufacturer installs on the user's device, a software pre-programmed to execute a particular algorithm that combines a particular set of biomarkers. This option is most convenient for end users who prefer the diagnosis to be based on a specific biomarker sets.
(3) The Dynamic Database option is similar to the UDR option, except that the algorithm is programmed into a central data management system, such as a LIS, instead of the device itself. A user, e.g., a device manufacturer engineer or a physician or laboratorian, can program the LIS and use the algorithm for OSA diagnosis. LIS can be combined with various automation systems, and other database storing patient results to provide timely and accurate diagnosis for OSA.

The scope of the present invention is as set forth in the appended claims rather than being limited to the examples contained in the forgoing description.

### EXAMPLES

The following examples are offered to illustrate, but not to limit the claimed invention.

### EXAMPLE 1 SELECTING BIOMARKERS

A multicenter prospective trial was conducted enrolling 128 patients with suspected OSA as well as a control group of healthy individuals who do not have OSA. A group of biomarkers, including HbAlc, CRP, IL-6, uric acid, EPO, were tested by personnel blinded to patient characteristics. All subjects underwent a diagnostic sleep study (polysomnography).. Patients and control group's AHI, minimum oxygen saturation, BMI and ESS, and other standard clinical assessment for OSA were measured. The diagnosis of the presence and the severity of OSA of each patient were accordingly made. Clinicians were not provided with biomarker results prior to patient diagnosis.

Table 2 shows the clinician's diagnosis of the 128 patients: 26 were diagnosed with moderate to severe OSA; 21 were diagnosed as having mild OSA; and 23 were diagnosed as having no OSA.

Correlations between the clinical diagnosis and the single biomarker diagnosis using the statistical analysis disclosed herein were shown in Table 3. Among the biomarkers, HbAlc, CRP, Uric Acid, and IL-6 showed the strongest association with clinical symptoms of OSA.

A Receiver Operating Characteristic (ROC) curve analysis of results from the 70 male subjects in the study was performed to assess the performance of diagnosis tests for OSA using each one of these biomarkers. See Table 4. The AUCs of these markers range from 0.52 and 0.76. HbAlc and CRP showed the highest of AUCs of the tested biomarkers, 0.74 and 0.75, respectively. See Table 3. Figures 1A-1C show that HbAlc levels and CRP levels can separate subjects not having OSA or only mild OSA from those having moderate to severe OSA; subjects having moderate to severe OSA have on average significantly higher HbAlc and CRP levels, respectively. Areas Under the Curve (AUCs) for diagnosis of moderate/severe OSA were >0.70 for HbAlc and CRP (p<0.001), indicating these two biomarkers can be used for OSA diagnosis.

Figures 1A and 1B illustrate that measuring HbAlc and CRP levels, respectively, are effective in distinguishing subjects having no OSA/mild OSA from subjects having moderate/severe OSA subjects. AUCs were greater than 0.60 for uric acid, IL-6, and EPO. Many of the moderate/severe OSA subjects were pre-diabetic (HbAlc ≥ 5.7%), with high cardiovascular risk (CRP > 0.3). It was also observed that individual biomarkers performed better or worse in specific clinical subgroups, e.g. HbA1c achieved significant group separation in obese subjects (p<0.05), as did CRP in non-obese subjects (p<0.01).

**Table 2. Patient population in the study.**

| | Male | Female | TOTAL |
|---|---|---|---|
| Control | 22 | 36 | 58 |
| Non-OSA | 10 | 13 | 23 |
| OSA Mild | 15 | 6 | 21 |
| OSA Moderate | 6 | 1 | 7 |
| OSA Severe | 17 | 2 | 19 |
| TOTAL | 70 | 58 | 128 |

**Table 3. Correlations of biomarkers to clinical measures.**

| | AHI | Min O2 | BMI | ESS |
|---|---|---|---|---|
| Alc | 0.46 | -0.44 | 0.37 | -0.23 |
| CRP | 0.56 | -0.35 | 0.60 | -0.08 |
| Uric acid | 0.30 | -0.11 | 0.52 | 0.06 |
| IL-6 | 0.44 | -0.29 | 0.35 | -0.18 |
| EPO | 0.24 | -0.17 | 0.00 | 0.04 |

**Table 4. Individual marker's performance in diagnosing OSA.**

| Test | Area |
|---|---|
| BMI | 0.76 |
| HbAlc (%) | 0.74 |
| CRP (mg/dL) | 0.75 |
| Uric Acid (mg/dL) | 0.61 |
| IL-6 (pg/mL) | 0.66 |
| EPO (miU/mL) | 0.63 |

### EXAMPLE 2: USING ALGORITHMS OF COMBINATIONS OF BIOMARKERS TO FOR OSA DIAGNOSIS.

This example shows that algorithms combining of two to three biomarkers -to produce a multimarker index for these biomarkers - can be used for accurately diagnosing OSA. A multimarker index produced for a subject using any one of these algorithms can be used as an aid in the diagnosis of OSA in conjunction with polysomnography (sleep study) findings and clinical signs and symptoms.

During the algorithm development process, the discriminative power of a group of 5 biomarkers (HbAlc, CRP, IL-6, uric acid, EPO) were investigated. Algorithms using 2- and 3-biomarker combinations in the group were developed and optimized using Linear Model and Non-Linear Model, and 4 optimization methods: Simulated Annealing (http://en.wikipedia.org/wiki/Simulated_annealing), Pattern Search (http://en.wikipedia.org/wiki/Pattern_search_(optimization)), Brute Force, and Genetic Algorithm (http://en.wikipedia.org/wiki/Genetic_algorithm). Linear values, i.e., the original levels of the biomarkers, or log values, i.e., the logarithmic values of the levels of the biomarkers were used in the algorithms. A set of algorithms were generated using various biomarker combinations and mathematical models. The algorithms' AUC, specificity/sensitivity were examined and top performing algorithms are presented in Table 5.

Table 5 shows several algorithms of the combinations significantly improved the diagnosis accuracy compared to individual biomarkers. For example, a "Linear Model - Log Value - 3 Marker" combination of HbAlc, CRP, and EPO yielded an 8-point increase in AUC (0.84) over individual markers (0.76). The diagnosis method using the algorithm has a high sensitivity and specificity: the specificity is 81% when the sensitivity is 80%; and the specificity is 79% when the sensitivity is 85% in the diagnosis of moderate/severe OSA. The multimarker index can be calculated according to this algorithm using the equation: 12.8117*log(HbA1c) + 0.74983*log(CRP) + 1.53056*log(EPO).

**Table 5. Algorithms using combinations of biomarkers and mathematical classification models**

| **Setup** | **Markers** | **All Features** | **Weight Array** | **AUC (95% CI)** | **If Sensitivity ≈80%, then Specificity is:** | **If Sensitivity** ≈**85%, then Specificity is:** |
|---|---|---|---|---|---|---|
| Linear | HbA1c, | HbAlc, CRP | 1.1317 | 0.80 | 79% | 51% |
| Model-Linear Value - 2 Markers (Positive) | CRP | | 1.0895 | (0.69-0.91) | | |
| Non-linear Model - Linear Value - 2 Markers (Negative) | HbA1c, CRP | HbA1c, CRP, HbA1c*HbA 1c, HbA1c*CRP , CRP*CRP | 0.78221 - 0.74132 - 0.11822 0.073841 -0.10699 | 0.81 (0.70-0.92) | 79% | 51% |
| Non-linear Model-Linear Value - 2 Markers (Negative) | HbA1c, IL-6 | HbAlc, IL-6, HbA1c*HbA 1c, HbA1c*IL-6, IL-6*IL-6 | 1.5832 0.25718 - 0.16651 - 0.07381 0.018817 | 0.81 (0.71-0.91) | 60% | 60% |
| Linear Model-Log Value - 2 Markers (Negative) | HbA1c, CRP | HbA1c, CRP | -3.8543 - 0.21863 | 0.80 (0.69-0.91) | 66% | 60% |
| Non-linear Model-Log Value - 2 Markers (Negative) | HbA1c, CRP | HbA1c, CRP, HbA1c*HbA 1c, HbA1c*CRP , CRP*CRP | -2.7413 - 0.1372 - 1.1671 - 0.0927 0.0123 | 0.81 (0.70-0.92) | 66% | 60% |
| Linear Model - Linear Value - 3 Markers (Positive) | HbA1c, CRP, EPO | HbA1c, CRP, EPO | 2.2809 2.2273 0 .052657 | 0.83 (0.72-0.93) | 85% | 85% |
| Linear Model-Linear Value - 3 Markers (Negative) | HbA1c, CRP, Uric Acid | HbAlc, CRP, Uric Acid | - 2.2031 - 1.9753 - 0.50476 | 0.81 (0.70-0.93) | 81% | 77% |
| Linear Model-Linear Value - 3 Markers (Positive) | HbA1c, Uric Acid, EPO | HbA1c, Uric Acid, EPO | 2.1466 0.42874 0.13984 | 0.82 (0.71-0.93) | 83% | 79% |
| Non-linear Model-Linear Value - 3 Markers (Positive) | HbA1c, CRP, IL-6 | HbA1c, CRP, IL-6, HbA1c*HbA 1c, HbA1c*CRP , HbA1c*IL-6, CRP*CRP, CRP*IL-6, IL-6*IL-6 | -0.2878 0.40799 -0.14152 0.064756 0.056639 0.066875 0.56735 - 0.3234 -0.024054 | 0.85 (0.75-0.94) | 72% | 60% |
| Non-linear Model - Linear Value - 3 Markers (Negative) | HbA1c, IL-6, EPO | HbA1c, IL-6, EPO, HbA1c*HbA 1c, HbA1c*IL-6, HbA1c*EPO , IL-6*IL-6, IL-6*EPO, EPO*EPO | -0.6826 0.27396 0.06468 0.038385 -0.050789 0.01218 0.021173 - 0.023076 - 0.0056012 | 0.87 (0.78-0.96) | 94% | 62% |
| Linear Model - Log Value - 3 Markers (Positive) | HbA1c, CRP, EPO | HbA1c, CRP, EPO | 12.8117 0.74983 1.53056 | 0.84 (0.75-0.94) | 81% | 79% |
| Linear Model - Log Value - 3 Markers (Positive) | HbA1c, CRP, Uric Acid | HbAlc, CRP, Uric Acid | 10.9394 0.437857 1.72504 | 0.83 (0.72-0.94) | 77% | 75% |
| Non-linear Model - Linear Value - 3 Markers (Negative) | HbA1c, CRP, EPO | HbA1c, CRP, EPO, HbA1c*HbA 1c, HbA1c*CRP , HbA1c*EPO , CRP*CRP, CRP*EPO, EPO*EPO | -1.2699 1.3237 -0.064941 0.054106 -0.12616 0.03781 0.20439 -0.15904 - 0.0066174 | 0.86 (0.75-0.96) | 92% | 89% |

| | | | | | | |
|---|---|---|---|---|---|---|
| Note: - "Markers" column includes the biomarkers that are used in the corresponding algorithm. - "positive" indicates the algorithm is positive logic. "negative" indicates that the algorithm is negative logic. - "Setup" column includes 3 pieces of information. 1) Algorithm model type, e.g. linear or non-linear. If a model is non-linear, cross terms, in addition to biomarker itself, will be used in the multimarker index, e.g. HbA1c*CRP. 2) How the value of biomarker is used in the multimarker index. If it is "Linear Value", the value of biomarker is used directly. If it is "Log Value", the logarithmic value of biomarker is used in the formula. 3) How many biomarkers are used in the multimarker index. - "All Features" column indicates all the terms that are used in the formula of the multimarker index. If the algorithm model is linear, "All Features" column is same as "Markers" column. If the algorithm model is non-linear, "All Features" column include cross terms as well as the ones in "Markers" column. - "Weight Array" is an algorithm. Each includes weight/coefficient of each term in "All Features" column for constructing the multimarker index. For example: the 5th algorithm (shown as below) is non-linear model and log value based on HbAlc and CRP. Since it is a non-linear model, cross terms are used. Therefore, HbA1c*HbA1c, HbA1c*CRP, and CRP*CRP show up in "All Features" column. Since it is based log value, the final formula of the multimarker index is equal to 1.7328*log(HbA1c) + 0.93802*log(CRP)-0.17974*log(HbA1c)*log(HbA1c)-0.16968*log(HbA1c)*log(CRP)-0.31994*log(CRP)*log(CRP). - "AUC (95% CI)" column: the number in the top line is the AUC value. The two numbers in the parentheses in the bottom line indicate the AUC range of 95% confidence level. | | | | | | |

Not only the algorithms of combinations of biomarkers outperform individual biomarkers in diagnosing OSA, they also outperform most clinical measurements. For example, a "Linear Model - Log Value - 3 Marker" combination of HbAlc, CRP, and EPO has an AUC value of 0.84. It is not only higher than the AUCs of all individual biomarkers: HbAlc (0.76), CRP (0.73), IL-6 (0.65), EPO (0.65) and Uric Acid (0.61), but also higher than the AUCs of the clinical measurements: BMI (0.76), Age (0.63), Diastolic blood pressure (Diastolic BP) (0.63) and Systolic blood pressure (Systolic BP) (0.58), Epworth Sleepiness Scale (0.52), and mean O₂ saturation (0.80) (Table 6).

**Table 6. Comparison of combinations of biomarkers to individual biomarkers, clinical Measures and polysomnography.**

| | | **AUC** | **95% CI** |
|---|---|---|---|
| Biomarkers | Biomarker Index (Linear Model - Log Value - HbAlc CRP, EPO) | 0.84 | 0.75 - 0.94 |
| | HbAlc (%) | 0.76 | 0.64 - 0.88 |
| | CRP (mg/dL) | 0.73 | 0.60 - 0.85 |
| | IL-6 (pg/mL) | 0.65 | 0.52 - 0.78 |
| | EPO (mIU/mL) | 0.65 | 0.51 - 0.78 |
| | Uric Acid (mg/dL) | 0.61 | 0.47 - 0.75 |
| Clinical Measures | BMI | 0.76 | 0.64 - 0.87 |
| | Age | 0.63 | 0.50 - 0.76 |
| | Diastolic BP | 0.63 | 0.46 - 0.80 |
| | Systolic BP | 0.58 | 0.41 - 0.76 |
| | Epworth Sleepiness Scale | 0.52 | 0.36 - 0.68 |
| Polysomnography | AHI | 1.00 | 1.00 - 1.00 |
| | Minimum O₂ Saturation | 0.95 | 0.90 - 1.00 |
| | Average O₂ Saturation | 0.80 | 0.68 - 0.92 |

In addition, the combinations of the biomarker can be used to distinguish the OSA of different severity. Figure 4A shows that HbAlc levels were significantly higher in patients with moderate/severe OSA than in controls (p<0.001), as were CRP (p<0.001), EPO (p<0.05), and the "Linear Model - Log Value - 3 Marker" combination of three biomarkers (HbAlc, hsCRP, EPO) (p<0.0001). These findings were observed in lean (BMI <30) as well as obese (BMI ≥30) patients; values in moderate/severe OSA patients were higher than controls in both lean and obese groups. Figure 4B shows that the "Linear Model - Log Value - 3 Marker" combination of HbAlc, CRP, and EPO was able to separate subjects having non-OSA, mild OSA, moderate OSA, and severe OSA base on their multimarker indices based on the algorithm of the combination. Subjects having more severe forms of OSA in general have higher multimarker indices than subjects having milder forms of OSA. See Figure 4B.

## Claims

1. A method of predicting or diagnosing obstructive sleep apnea (OSA) in a subject, the method comprising:
a. measuring the level of HbA1c and at least one biomarker selected from CRP, IL-6, uric acid and erythropoietin (EPO) in a sample from the subject
b. predicting or diagnosing OSA in the subject if the measured level of the biomarkers selected in a) are elevated.

2. The method of claim 1 wherein a measured level of HbAlc equal to or higher than 5.7% predicts or diagnoses OSA.

3. The method of any one of claims 1 to 2 wherein at least one of the at least two biomarkers is CRP.

4. The method of any one of claims 1 to 3 wherein at least one of the at least two biomarkers is CRP and a measured level equal to or higher than 0.2 mg/dL predicts or diagnoses OSA.

5. The method of any one of claims 1 to 4, the method comprising:
a. measuring the level of HbA1c and at least two biomarkers selected from CRP, IL-6, uric acid and erythropoietin (EPO) in a sample from the subject
b. predicting or diagnosing OSA in the subject if the measured level of the three biomarkers selected in a) are elevated.

6. The method of claim 5 wherein the three biomarkers are HbA1c, CRP and EPO.

7. The method of any one of claims 1 to 6 wherein the diagnosis is moderate or severe OSA.

8. The method of any one of claims 1 to 7 wherein elevated levels are determined by comparison with a reference value.

9. A method of monitoring whether a therapy is effective for treating OSA, comprising the steps of:
a. measuring the levels of HbAlc and at least one or two biomarkers in a sample taken from a patient before therapy, wherein the at least one or two biomarkers are selected from the group of CRP, IL-6, uric acid, and EPO;
b. measuring the levels of the at least two or three biomarkers in a sample taken from a patient after the therapy, wherein the therapy is considered effective if the levels of the at least two or three biomarkers are decreased compared to the levels measured in step a.

10. A method of monitoring whether a therapy is effective for treating OSA, comprising the steps of:
a. measuring the levels of HbAlc and at least one or two biomarkers in a sample taken from a patient after therapy, wherein the at least one or two biomarkers are selected from the group of CRP, IL-6, uric acid, and EPO;
b. comparing the measured levels of the at least two or three biomarkers against a predetermined reference value, wherein the therapy is considered effective if the levels of the at least two or three biomarkers are lower than the predetermined reference value.

11. The method of any one of claims 1 to 10 wherein the biomarkers are a combination of two or three biomarkers selected from the combinations in Table 5.

12. The method of any one of claims 1 to 11 to wherein the sample is a blood sample,

13. The method of claim 12 wherein the blood sample is a plasma or serum sample.

14. A non-transitory computer readable medium having computer-executable instructions which, when executed, causes a processor to perform a method according to any one of claims 1 to 13.

15. A computer system for diagnosing OSA comprising:
a. a detection device configured to detect HbAlc and one or one or more biomarkers that are selected from the group consisting of CRP, IL-6, uric acid, and EPO; and
b. an analysing device comprising one or more processors and a non-transitory computer readable medium according to claim 14.

## Patentansprüche

1. Verfahren zur Vorhersage oder zur Diagnose von obstruktiver Schlafapnoe (OSA) in einer Person, wobei das Verfahren umfasst:
a. Messen des Spiegels von HbA1c und mindestens einem Biomarker ausgewählt aus CRP, IL-6, Harnsäure und Erythropoetin (EPO) in einer von der Person erhaltenen Probe
b. Vorhersagen oder Diagnostizieren von OSA für die Person, wenn die gemessenen Spiegel der Biomarker, welchen in a) ausgewählt wurden, erhöht sind.

2. Verfahren nach Anspruch 1, worin ein gemessener Spiegel von HbA1c, der gleich oder höher als 5,7% ist, OSA vorhersagt oder diagnostiziert.

3. Verfahren nach einem der Ansprüche 1 bis 2, worin mindestens einer der mindestens zwei Biomarker CRP ist.

4. Verfahren nach einem der Ansprüche 1 bis 3, worin mindestens einer der mindestens zwei Biomarker CRP ist und ein gemessener Spiegel, der gleich oder höher als 0,2 mg/dL ist, OSA vorhersagt oder diagnostiziert.

5. Verfahren nach einem der Ansprüche 1 bis 4, wobei das Verfahren umfasst:
a. Messen des Spiegels von HbA1c und von mindestens zwei Biomarkern ausgewählt aus CRP, IL-6, Harnsäure und Erythropoetin (EPO) in einer von der Person erhaltenen Probe
b. Vorhersagen oder Diagnostizieren von OSA für die Person, wenn die gemessenen Werte der drei Biomarker, welche in a) ausgewählt wurden, erhöht sind.

6. Verfahren nach Anspruch 5, worin die drei Biomarker HbA1c, CRP und EPO sind.

7. Verfahren nach einem der Ansprüche 1 bis 6, worin die Diagnose moderate oder schwere OSA ist.

8. Verfahren nach einem der Ansprüche 1 bis 7, worin erhöhte Spiegel durch Vergleich mit einem Referenzwert bestimmt werden.

9. Verfahren zur Überwachung, ob eine Therapie zur Behandlung von OSA effektiv ist, umfassend die Schritte:
a. Messen der Spiegel von HbA1c und von mindestens einem oder zwei Biomarkern in einer von einem Patienten vor der Therapie entnommenen Probe, worin die mindestens ein oder zwei Biomarker ausgewählt ist aus der Gruppe von CRP, IL-6, Harnsäure und EPO;
b. Messen der Spiegel der mindestens zwei oder drei Biomarker in einer von einem Patienten nach der Therapie entnommenen Probe, worin die Therapie als wirksam anzusehen ist, wenn die Spiegel der mindestens zwei oder drei Biomarker im Vergleich zu den in Schritt a) gemessenen Spiegeln erniedrigt ist.

10. Verfahren zur Überwachung, ob eine Therapie zur Behandlung von OSA effektiv ist, umfassend die Schritte:
a. Messen der Spiegel von HbA1c und von mindestens einem oder zwei Biomarkern in einer von einem Patienten nach der Therapie entnommenen Probe, worin die mindestens ein oder zwei Biomarker ausgewählt ist aus der Gruppe von CRP, IL-6, Harnsäure und EPO;
b. Vergleichen der gemessenen Spiegel der mindestens zwei oder drei Biomarker zu einem vorbestimmten Referenzwert, wobei die Therapie als effektiv anzusehen ist, wenn die Spiegel der mindestens zwei oder drei Biomarker niedriger als der vorbestimmte Referenzwert sind.

11. Verfahren nach einem der Ansprüche 1 bis 10, worin die Biomarker eine Kombination von zwei oder drei Biomarkern ausgewählt aus den Kombinationen in Tabelle 5 sind.

12. Verfahren nach einem der Ansprüche 1 bis 11, worin die Probe eine Blutprobe ist.

13. Verfahren nach Anspruch 12, worin die Blutprobe eine Plasma- oder Serumprobe ist.

14. Ein nicht-vorübergehendes computerlesbares Medium mit computerausführbaren Instruktionen, welche, wenn ausgeführt, einen Prozessor veranlassen, ein Verfahren gemäß einem der Ansprüche 1 bis 13 durchzuführen.

15. Computersystem zur Diagnose von OSA umfassend.
a. ein Detektionsmittel, welches ausgestaltet ist, HbA1c und einen oder einen oder mehrere Biomarker ausgewählt aus der Gruppe bestehend aus CRP, IL-6, Harnsäure und EPO zu detektieren; und
b. eine Analyseeinheit umfassend einen oder mehrere Prozessoren und ein nicht-vorübergehendes computerlesbares Medium gemäß Anspruch 14.

## Revendications

1. Procédé de prédiction ou de diagnostic de l'apnée obstructive du sommeil (AOS) chez un sujet, le procédé comprenant :
a. la mesure du taux d'HbAlc et d'au moins un biomarqueur choisi parmi la CRP, l'IL-6, l'acide urique et l'érythropoiétine (EPO) dans un échantillon provenant du sujet
b. la prédiction ou le diagnostic de l'AOS chez le sujet si le taux mesuré des biomarqueurs choisis en a) est élevé.

2. Procédé selon la revendication 1 dans lequel un taux mesuré d'HbAlc supérieur ou égal à 5,7 % prédit ou diagnostique l'AOS.

3. Procédé selon l'une quelconque des revendications 1 à 2 dans lequel au moins un des au moins deux biomarqueurs est une CRP.

4. Procédé selon l'une quelconque des revendications 1 à 3 dans lequel au moins un des au moins deux biomarqueurs est une CRP et un taux mesuré supérieur ou égal à 0,2 mg/dl prédit ou diagnostique l'AOS.

5. Procédé selon l'une quelconque des revendications 1 à 4, le procédé comprenant :
a. la mesure du taux d'HbAlc et d'au moins deux biomarqueurs choisis parmi la CRP, l'IL-6, l'acide urique et l'érythropoiétine (EPO) dans un échantillon provenant du sujet
b. la prédiction ou le diagnostic de l'AOS chez le sujet si le taux mesuré des trois biomarqueurs choisis en a) est élevé.

6. Procédé selon la revendication 5 dans lequel les trois biomarqueurs sont l'HbA1c, la CRP et l'EPO.

7. Procédé selon l'une quelconque des revendications 1 à 6 dans lequel le diagnostic est une AOS modérée ou sévère.

8. Procédé selon l'une quelconque des revendications 1 à 7 dans lequel les taux élevés sont déterminés par comparaison avec une valeur de référence.

9. Procédé pour surveiller si une thérapie est efficace pour traiter l'AOS, comprenant les étapes de :
a. mesure des taux d'HbAlc et d'au moins un ou deux biomarqueurs dans un échantillon prélevé auprès d'un patient avant une thérapie, dans lequel les au moins un ou deux biomarqueurs sont choisis dans le groupe constitué de la CRP, de l'IL-6, de l'acide urique et de l'EPO ;
b. mesure des taux des au moins deux ou trois biomarqueurs dans un échantillon prélevé auprès d'un patient après la thérapie, dans lequel la thérapie est considérée efficace si les taux des au moins deux ou trois biomarqueurs sont réduits comparativement aux taux mesurés à l'étape a.

10. Procédé pour surveiller si une thérapie est efficace pour traiter l'AOS, comprenant les étapes de :
a. mesure des taux d'HbAlc et d'au moins un ou deux biomarqueurs dans un échantillon prélevé auprès d'un patient après la thérapie, dans lequel les au moins un ou deux biomarqueurs sont choisis dans le groupe constitué de la CRP, de l'IL-6, de l'acide urique et de l'EPO ;
b. comparaison des taux mesurés des au moins deux ou trois biomarqueurs par rapport à une valeur de référence prédéterminée, dans lequel la thérapie est considérée efficace si les taux des au moins deux ou trois biomarqueurs sont inférieurs à la valeur de référence prédéterminée.

11. Procédé selon l'une quelconque des revendications 1 à 10 dans lequel les biomarqueurs sont une combinaison de deux ou trois biomarqueurs choisis parmi les combinaisons dans le Tableau 5.

12. Procédé selon l'une quelconque des revendications 1 à 11 dans lequel l'échantillon est un échantillon sanguin.

13. Procédé selon la revendication 12 dans lequel l'échantillon sanguin est un échantillon de plasma ou de sérum.

14. Support lisible par ordinateur non transitoire ayant des instructions exécutables par un ordinateur qui, lorsqu'elles sont exécutées, amène un processeur à réaliser un procédé selon l'une quelconque des revendications 1 à 13.

15. Système informatique pour le diagnostic de l'AOS comprenant :
a. un dispositif de détection configuré pour détecter l'HbA1c et un ou un ou plusieurs biomarqueurs qui sont choisis dans le groupe constitué de la CRP, de l'IL-6, de l'acide urique et de l'EPO ; et
b. un dispositif d'analyse comprenant un ou plusieurs processeurs et un support lisible par ordinateur non transitoire selon la revendication 14.
